(19) Europäisches Patentamt
European Patent Office
Office européen des brevets

(11) Veröffentlichungsnummer: **0 232 826 B1**

(12) **EUROPÄISCHE PATENTSCHRIFT**

(45) Veröffentlichungstag der Patentschrift: **06.05.92**

(51) Int. Cl.5: **C07C 47/228**, C07C 45/51, C07C 43/166, C07C 41/32, C07C 41/16

(21) Anmeldenummer: **87101391.8**

(22) Anmeldetag: **02.02.87**

# (54) Verfahren zur Herstellung eines Aldehydes.

(30) Priorität: **14.02.86 CH 598/86**
**27.11.86 CH 4742/86**

(43) Veröffentlichungstag der Anmeldung:
**19.08.87 Patentblatt 87/34**

(45) Bekanntmachung des Hinweises auf die Patenterteilung:
**06.05.92 Patentblatt 92/19**

(84) Benannte Vertragsstaaten:
**AT BE CH DE FR GB IT LI NL**

(56) Entgegenhaltungen:
**US-A- 2 477 312**

**CHEMICAL ABSTRACTS, Band 94, Nr. 5, 2. Februar 1981, Seite 528, Zusammenfassung Nr. 30299n, Columbus, Ohio, US; C.F. GARBERS et al.: "Electrophilic addition of a-chloromethyl methyl ether to alkenylarenes. Synthesis of cyclamen aldehyde"**

(73) Patentinhaber: **L. GIVAUDAN & CIE Société Anonyme**

**CH-1214 Vernier-Genève(CH)**

(72) Erfinder: **Gygax, Peter, Dr.**
**Weiherhof 39**
**CH-8604 Volketswil(CH)**

(74) Vertreter: **Urech, Peter, Dr. et al**
**Grenzacherstrasse 124 Postfach 3255**
**CH-4002 Basel(CH)**

**EP 0 232 826 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung eines Aldehydes, und zwar des p-tert. Butyl-α-methyl-hydrozimtaldehydes

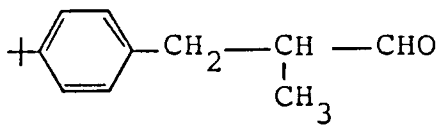

I

eines bekannten Riechstoffes.

Das Verfahren ist dadurch gekennzeichnet, dass man den p-tert. Butylbenzyl-propenyläther unter der Einwirkung eines Cu(1) oder Cu(2)-Halogenides und einer Base bei Temperaturen von ca 130 ° C bis 300 ° C zu p-tert.-Butyl-α-methyl-hydrozimtaldehyd umlagert.

Dieser Aether (II) ist eine neue Verbindung und bildet ebenfalls Gegenstand der vorliegenden Erfindung. Er kann als E- oder Z-Isomeres oder als Gemisch der beiden Isomeren vorliegen.

Der Aether (II) ist herstellbar durch katalytische Isomerisierung des p-tert. Butylbenzyl-allyläthers (III).

Auch dieser Aether (III) ist eine neue Verbindung.

Er ist herstellbar durch Verätherung des bekannten p-tert. Butylbenzylchlorids (IV).

Der Zugang zu I kann wie folgt dargestellt werden:

<u>Schema</u>

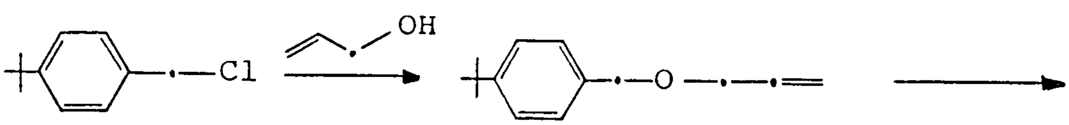

IV III

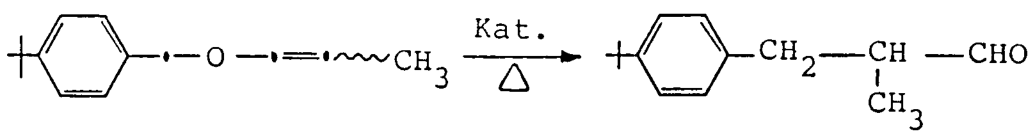


II I

Als Katalysatoren zur Umlagerung von II werden Cu-Halogenide, und zwar $Cu^{1+}$ - oder $Cu^{2+}$-Halogenide, insbesondere aber $Cu^{1+}$-Halogenide verwendet, und zwar das Chlorid, Bromid oder Jodid. Bevorzugt ist das Jodid, gefolgt vom Bromid.

Aber auch Gemische von $Cu^{1+}/Cu^{2+}$-Halogeniden in beliebigem Mischverhältnis, z.B. 1:100 bis 100:1, können verwendet werden.

Es können die wasserfreien oder die kristallwasserhaltigen Salze eingesetzt werden; bevorzugt sind die ersteren.

Die Mengen sind, wie gesagt, katalytisch, also z.B. ca. 1 bis 20, insbesondere ca. 2 bis 15 Gew.% der Verbindung II.

Der Temperaturbereich ist derjenige von ca. 130 ° C bis 300 ° C, insbesondere derjenige von ca. 135 ° C bis 250 ° C. Speziell bevorzugt sind ca. 180 ° C bis 200 ° C.

Eine geringe Menge, also z.B. 1 bis 5 Gew.% einer Base wird erfindungsgemäss zugesetzt. Geeignet sind hierzu insbesondere Carbonate und Bicarbonate, insbesondere Alkalimetallcarbonate und Alkalimetallbicarbonate, z.B. $Na_2CO_3$, $NaHCO_3$, etc.

Die Reaktion kann in An- oder Abwesenheit von Lösungsmitteln durchgeführt werden. Als Lösungsmittel geeignet sind insbesondere hochsiedende inerte Lösungsmittel, z.B. Paraffinöl, tert. Butyltoluol, hochsiedende Aether wie Diphenyläther, etc. Die Menge Lösungsmittel ist nicht kritisch.

Bevorzugt wird aber ohne Lösungsmittel gearbeitet.

Die Reaktion kann durch Zugabe einer geringen Menge, z.B. 0,5 - 2 Gew.% eines Alkalijodids, z.B. Natrium- oder Kaliumjodid beschleunigt werden.

Die katalytische Isomerisierung der Doppelbindung (Verbindung III→ Verbindung II) kann nach für diesen Zweck an sich bekannten Methoden, also nach Methoden zur Herstellung von Enoläthan aus Alkyläthan durchgeführt werden, siehe z.B. Houben-Weyl, Methoden der organischen Chemie, Bd. 5/lb, (1972), Seiten 638 - 671.

Es kommt hierzu heterogene oder homogene Katalyse in Betracht; die zweckmässigen Parameter sind die folgenden:

Tabelle I

| | | | |
|---|---|---|---|
| heterogene Katalyse (bevorzugt) | | | |
| Edelmetalle, z.B. Ru, Pd, Rh, Pt; aber auch weitere Metalle, z.B. Ni, Co, Cu, Mo | Träger z.B. Aluminiumoxid, Aktivkohle, Kieselgel | Temperatur z.B. ca. 120 - 250°C, bevorzugt ca. 140 - 200°C | Lösungsmittel fakultativ, insbesondere hochsiedende inerte Lösungsmittel, wie Kohlenwasserstoffe, Aether, Aromaten |
| homogene Katalyse | | | |
| Edelmetall(komplex)-verbindungen, z.B. tris(Triphenylphosphin)-Rutheniumdichlorid, bis-(Triphenylphosphin)-Rhodiumchlorid | | ca. 100 - 150°C, bevorzugt ca. 80 - 110°C | Lösungsmittel fakultativ, insbesondere hochsiedende inerte Lösungsmittel, wie Kohlenwasserstoffe, Aether, Aromaten |

| | | |
|---|---|---|
| Iridiumkatalysatoren, z.B. $[Ir(cod)(PMePh_2)_2]PF_6$ (cod = cyclooctadien) | ca. 20°C | Aether, insbesondere Tetrahydrofuran |
| Metallcarbonyle, wie z.B. Eisen-, Molybdän- oder Wolframcarbonyle | ca. 50 - 200°C | Lösungsmittel fakultativ, insbesondere hochsiedende, inerte Lösungsmittel, wie Kohlenwasserstoffe, Aether, Aromaten |

Die Verätherung des Chlorids IV erfolgt zweckmässigerweise nach an sich bekannten Verfahren, also insbesondere unter der Einwirkung von Basen, siehe z.B. Houben-Weyl, Methoden der organischen Chemie, Band 6/3, (1965), Seite 24 ff.

Das stöchiometrische Verhältnis von Chlorid IV zu Allylalkohol beträgt zweckmässigerweise ca. 1:1; ein Ueberschuss von Allylalkohol wirkt sich nicht nachteilig aus, ist aber eher unwirtschaftlich.

Als Basen geeignet sind insbesondere Alkalimetallhydroxide, Erdalkalimetallhydroxide, Alkalimetallcarbonate, Erdalkalimetallcarbonate, Alkalimetallbicarbonate, Erdalkalimetallbicarbonate, etc.

Die Base wird zweckmässigerweise in mindestens stöchiometrischer Menge eingesetzt. Ein mässiger, z.B. 10 bis 50%-iger Ueberschuss ist aber nicht nachteilig.

Man kann in Anwesenheit oder Abwesenheit von Lösungsmitteln arbeiten. Zweckmässige Lösungsmittel sind Alkane, Aromaten, Aether. Vorzugsweise arbeitet man aber ohne Lösungsmittel.

Die Reaktionstemperatur liegt beispielsweise zwischen ca. 30 und 100 °C, vorzugsweise zwischen ca. 40 und 90 °C. Optimal ist der Bereich von ca. 60 bis 70 °C.

Es ist vorteilhaft, in Anwesenheit von Phasentransferkatalysatoren zu arbeiten. Als Phasentransferkatalysatoren kommen insbesondere die üblichen tetrasubstituierten Ammoniumsalze in Betracht, siehe z.B. E.V. Dehmlow, Phase transfer catalysis, Verlag Chemie, (1983), Seite 104 ff.

Auch Kronenäther - siehe z.B. C.L. Liotta, The chemistry of ethers (Editor: S. Patai), Suppl. F, Seite 157 ff. - sind gut geeignet, insbesondere in Anwesenheit von Alkalimetallcarbonaten, z.B. Natrium- oder Kaliumcarbonat, in Lösungsmitteln wie Aceton, wobei man in einem wasserfreien System arbeitet, siehe z.B. M. Makosza et al., J. Org. Chem. Vol. 43, (1978), Seite 4682.

Es ist vorteilhaft, die Umsetzungen III→ II und II→ I in Inertatmosphäre, also z.B. unter Stickstoff durchzuführen.

Beispiel

a) 88g (1,1 Mol) 50%-ige Natronlauge werden mit 4,6 g (0,016 Mol) Tetrabutylammoniumchlorid versetzt. Bei einer Temperatur von 35 °C wird innert ca. 20 Minuten ein Gemisch von 182,5 g (1,0 Mol) p-tert.-Butylbenzylchlorid und 61,5 g (1,06 Mol) Allylalkohol unter gutem Rühren zugetropft. Die Temperatur wird bei ≦ 70 °C gehalten, was nötigenfalls durch leichtes Kühlen bewerkstelligt wird. Es wird noch 12 Stunden bei 70 °C gut weitergerührt. Nach dem Abkühlen wird das Reaktionsgemisch 2 mal mit verdünnter Salzsäure gewaschen. Es fallen 204 g praktisch reiner p-tert.-Butylbenzyl-allyläther an. Die Substanz kann ohne Destillation für die nächste Stufe verwendet werden.

Sdp. des destillierten Materials: ca. 90° (13,33 Pa; 0,1 Torr).

b) 204 g (1,0 Mol) p-tert.-Butylbenzyl-allyläther werden mit 2,04 g Ruthenium auf Aluminiumoxid (5 %-ig) versetzt und unter Stickstoffatmosphäre 1 Stunde bei einer Badtemperatur von 180 °C gerührt. Nach dem Abfiltrieren des Katalysators wird am Hochvakuum destilliert. Man erhält 163,4 g p-tert.-Butylbenzyl-propenyläther als Gemisch des Z- und des E-Isomeren (80,1 % der Theorie bezogen auf p-tert.-Butylbenzyl-chlorid).

Sdp.: 140 - 145 °C.

c) 15,3 g Kupfer$^{1+}$ jodid, 2,55 g Natriumcarbonat und 1,02 g Natriumjodid werden unter Stickstoff in einem Kolben mit Tropftrichter, Thermometer und Rührer bei 185 °C vorgelegt. 101,8 g (0,49 Mol) p-tert.-Butylbenzyl-propenyläther (Isomerengemisch) werden innert 13 Minuten zugetropft. Nach weiteren 10 Minuten Rühren bei 185 - 190 °C wird die Heizung entfernt und nach dem Abkühlen das Reaktionsgemisch durch Filtration vom Katalysator abgetrennt. Die Destillation am Hochvakuum ergibt 69,4 g (68,2% der Theorie) reinen p-tert.-Butyl-α-methyl-hydrozimtaldehyd, Sdp. 110 °C.

$c_2$) Analoge Resultate ergeben sich bei Verwendung:
eines Gemisches von $Cu^{1+}/Cu^{2+}$-bromid (1:1),
eines Gemisches von $Cu^{1+}/Cu^{2+}$-chlorid (1:1), bei Verwendung von
CuBr, bei Verwendung von
CuCl,
$CuBr_2$,
$CuCl_2$,
beim Arbeiten in Paraffinöl (vom Siedepunkt >200 °C),
beim Arbeiten bei 160° oder bei 200 °C,
oder bei Halbierung der Gewichtsmenge des Katalysators.

## Patentansprüche

### Patentansprüche für folgende Vertragsstaaten : BE, CH, DE, FR, GB, IT, LI, NL

1. Verfahren zur Herstellung von p-tert.-Butyl-α-methylhydrozimtaldehyd, dadurch gekennzeichnet, dass man den p-tert.-Butylbenzyl-propenyläther unter der Einwirkung eines Cu(1) -oder Cu(2)-Halogenides und einer Base bei Temperaturen von ca. 130 ° C bis 300 ° C zu p-tert.-Butyl-α-methyl-hydrozimtaldehyd umlagert.

2. Verfahren nach Anspruch 1, dadurch gekennzeichnet, dass man als Katalysator ein Cu(1)-Halogenid, insbesondere Cu(1)-jodid verwendet.

3. Verfahren nach Anspruch 2, dadurch gekennzeichnet, dass man Cu(1)-bromid verwendet.

4. Verfahren nach Anspruch 1, dadurch gekennzeichnet, dass man als Katalysator ein Gemisch eines Cu-(1)- mit einem Cu(2)-Halogenid verwendet.

5. Verfahren nach einem der Ansprüche 1 bis 4, dadurch gekennzeichnet, dass man den p-tert. Butylbenzyl-propenyläther durch katalytische Isomerisierung des p-tert.-Butylbenzyl-allyläthers gewinnt.

6. Verfahren nach einem der Ansprüche 1 bis 4, dadurch gekennzeichnet, dass man den p-tert.-Butylbenzyl-allyläther durch Verätherung von p-tert.-Butylbenzylchlorid gewinnt.

7. p-tert.-Butylbenzyl-propenyläther.

### Patentansprüche für folgenden Vertragsstaat : AT

1. Verfahren zur Herstellung von p-tert.-Butyl-α-methylhydrozimtaldehyd, dadurch gekennzeichnet, dass man den p-tert.-Butylbenzyl-propenyläther unter der Einwirkung eines Cu(1)- oder Cu(2)-Halogenides und einer Base bei Temperaturen von ca. 130 ° C bis 300 ° C zu p-tert.-Butyl-α-methyl-hydrozimtaldehyd umlagert.

2. Verfahren nach Anspruch 1, dadurch gekennzeichnet, dass man als Katalysator ein Cu(1)-Halogenid, insbesondere Cu(1)-jodid verwendet.

3. Verfahren nach Anspruch 2, dadurch gekennzeichnet, dass man Cu(1)-bromid verwendet.

4. Verfahren nach Anspruch 1, dadurch gekennzeichnet, dass man als Katalysator ein Gemisch eines Cu-(1)- mit einem Cu(2)-Halogenid verwendet.

5. Verfahren nach einem der Ansprüche 1 bis 4, dadurch gekennzeichnet, dass man den p-tert. Butylbenzyl-propenyläther durch katalytische Isomerisierung des p-tert.-Butylbenzyl-allyläthers gewinnt.

6. Verfahren nach einem der Ansprüche 1 bis 4, dadurch gekennzeichnet, dass man den p-tert.-Butylbenzyl-allyläther durch Verätherung von p-tert.-Butylbenzylchlorid gewinnt.

## Claims

### Claims for the following Contracting States : BE, CH, DE, FR, GB, IT, LI, NL

1. A process for the manufacture of p-tert.butyl-α-methylhydrocinnamaldehyde, characterized by rearranging p-tert.-butylbenzyl propenyl ether to p-tert.butyl-α-methylhydrocinnamaldehyde under the influence of a Cu(1) or Cu(2) halide and a base at temperatures from about 130 ° C to 300 ° C.

2. A process according to claim 1, characterized in that a Cu(1) or (2) halide, preferably a Cu(1) halide, especially Cu(1) iodide, is used as the catalyst.

3. A process according to claim 2, characterized in that Cu(1) bromide is used.

4. A process according to claim 1, characterized in that a mixture of a Cu(1) halide with a Cu(2) halide is used as the catalyst.

5. A process according to any one of claims 1 to 4, characterized in that p-tert.-butylbenzyl propenyl ether is prepared by catalytically isomerizing p-tert.-butylbenzyl ally ether.

6. A process according to any one of claims 1 to 4, characterized in that the p-tert.-butylbenzyl ally ether is prepared by etherifying p-tert.-butylbenzyl chloride.

7. p-tert.-Butylbenzyl propenyl ether.

**Claims for the following Contracting State : AT**

1. A process for the manufacture of p-tert.butyl-α-methylhydrocinnamaldehyde, characterized by rearranging p-tert.-butylbenzyl propenyl ether to p-tert.butyl-α-methylhydrocinnamaldehyde under the influence of a Cu(1) or Cu(2) halide and a base at temperatures from about 130 ° C to 300 ° C.

2. A process according to claim 1, characterized in that a Cu(1) or (2) halide, preferably a Cu(1) halide, especially Cu(1) iodide, is used as the catalyst.

3. A process according to claim 2, characterized in that Cu(1) bromide is used.

4. A process according to claim 1, characterized in that a mixture of a Cu(1) halide with a Cu(2) halide is used as the catalyst.

5. A process according to any one of claims 1 to 4, characterized in that p-tert.-butylbenzyl propenyl ether is prepared by catalytically isomerizing p-tert.-butylbenzyl allyl ether.

6. A process according to any one of claims 1 to 4, characterized in that the p-tert.-butylbenzyl allyl ether is prepared by etherifying p-tert.-butylbenzyl chloride.

**Revendications**

**Revendications pour les Etats contractants suivants : BE, CH, DE, FR, GB, IT, LI, NL**

1. Procédé de préparation de p-tert-butyl-α-méthylhydrocinnamaldéhyde, caractérisé en ce qu'on transpose le p-tert-butylbenzyl-propényléther sous l'action d'un halogénure de Cu(1) ou de Cu(2) et d'une base à des températures allant d'environ 130 ° C à 300 ° C en p-tert-butyl-α-méthyl-hydrocinnamaldéhyde.

2. Procédé selon la revendication 1, caractérisé en ce qu'on utilise comme catalyseur un halogénure de Cu(1), en particulier l'iodure de Cu(1).

3. Procédé selon la revendication 2, caractérisé en ce qu'on utilise le bromure de Cu(1).

4. Procédé selon la revendication 1, caractérisé en ce qu'on utilise comme catalyseur un mélange d'un halogénure de Cu(1) avec un halogénure de Cu(2).

5. Procédé selon l'une des revendications 1 à 4, caractérisé en ce qu'on obbtient le p-tert-butylbenzyl-propényléther par isomérisation catalytique du p-tert-butyl-benzyl-allyléther.

6. Procédé selon l'une des revendications 1 à 4, caractérisé en ce qu on obtient le p-tert-butylbenzyl-allyléther par éthérification du chlorure de p-tert-butylbenzyle.

7. p-tert-butylbenzyl-propényléther.

**Revendications pour l'Etat contractant suivant : AT**

1. Procédé de préparation de p-tert-butyl-α-méthylhydrocinnamaldéhyde, caractérisé en ce qu'on transpose le p-tert-butylbenzyl-propényléther sous l'action d'un halogénure de Cu(1) ou de Cu(2) et d'une base à des températures allant d'environ 130 ° C à 300 ° C en p-tert-butyl-α-méthyl-hydrocinnamaldéhyde.

2. Procédé selon la revendication 1, caractérise en ce qu'on utilise comme catalyseur un halogénure de Cu(1), en particulier l'iodure de Cu(1).

3. Procédé selon la revendication 2, caractérisé en ce qu'on utilise le bromure de Cu(1).

4. Procédé selon la revendication 1, caractérisé en ce qu'on utilise comme catalyseur un mélange d'un halogénure de Cu(1) avec un halogénure de Cu(2).

5. Procédé selon l'une des revendications 1 à 4, caractérisé en ce qu'on obbtient le p-tert-butylbenzyl-propényléther par isomérisation catalytique du p-tert-butyl-benzyl-allyléther.

6. Procédé selon l'une des revendications 1 à 4, caractérisé en ce qu'on obtient le p-tert-butylbenzyl-allyléther par éthérification du chlorure de p-tert-butylbenzyle.